# EUROPÄISCHE PATENTSCHRIFT

(11) **EP 4 034 060 B1**
(45) Veröffentlichungstag und Bekanntmachung des Hinweises auf die Patenterteilung: **13.09.2023**
(21) Anmeldenummer: 19813226.8
(22) Anmeldetag: 25.09.2019
(51) Int. Cl.: A61F 13/00, A61F 13/08

(54) **BANDAGESYSTEM MIT ENTSTAUUNGSFUNKTION**
BANDAGE SYSTEM WITH DECONGESTIVE FUNCTION
SYSTÈME DE BANDAGE PRÉSENTANT UNE FONCTION DÉCONGESTIVE

(43) Veröffentlichungstag der Anmeldung: 03.08.2022
(73) Patentinhaber: Dr. Recknagel Gesundheitsservice GmbH, 10365 Berlin (DE); GPB Gesundheitszentrum Prenzlauer-Berg GmbH, 10407 Berlin (DE)
(72) Erfinder: VALDER, Uwe, 06295 Lutherstadt Eisleben (DE)
(74) Vertreter: Patentanwälte Schuster, Müller & Partner mbB
(86) Internationale Anmeldenummer: PCT/DE2019/100847
(87) Internationale Veröffentlichungsnummer: WO 2021/058043

(56) Entgegenhaltungen:
- EP-A1- 3 135 141
- EP-A2- 1 086 675
- WO-A1-2010/046130
- DE-A1-102018 107 576
- DE-U1-202006 006 884
- US-A- 2 702 998
- US-A1- 2005 267 393

## Beschreibung

### Stand der Technik

Die Erfindung geht aus von einem Bandagesystem mit Entstauungsfunktion zur Behandlung von Störungen des Lymph- und/oder Venensystems des menschlichen Körpers nach der Gattung des Oberbegriffs des Anspruchs 1.

Bandagesysteme, auch Orthesen genannt, sind in unterschiedlichsten Ausführungen und zu verschiedensten Anwendungen bekannt. Sie werden bei Venenerkrankungen und/oder Störungen des Lymphsystems, sog. Ödemen, sowie als Prophylaxe von Störungen des Venen- und/oder Lymphsystems bei ruhendem Körper sowie bei Gliedma-ßen in inaktiv liegender Stellung, beispielsweise als begleitende Therapie nach Operationen, angewendet.

Bekannt ist beispielsweise ein therapeutischer Strumpf, der einen Pressdruck gegen die Beine mit einem Druckprofil ausübt, das vom Knöchel bis zum oberen Oberschenkel abnimmt (DE 39 16 447 A1).

Bekannt ist ferner eine kompressive Orthese, insbesondere ein Kompressionsstrumpf, der wenigstens einen schlauchartigen Basisköper aus einem elastisch nachgebenden Grundmaterial aufweist, das elastisch isotrop mit durchgängig in Längsrichtung verlaufenden Einschnürungen ausgebildet ist. Zwischen den Einschnürungen verlaufen Längsrippen und ggf. Querrippen (DE 20 2006 006 884 U1).

Bei einem anderen Beinstrumpf zur Kompressionstherapie besteht das Gewebe aus einem aufgenoppten Flachstrick, der eine kombinierte Kompressions-Massagewirkung erzeugt (DE 20 2008 017 054 U1).

Bekannt ist auch eine Kompressionsmanschette zur humanmedizinischen Behandlung des Variocosesymptomkomplexes der menschlichen Beine, die aus einem Streifen aus einem verhältnismäßig dünnen hochelastischen Material besteht. An seiner Stoß- bzw. Überlappungsstelle wird der Streifen durch verstellbare Verschlüsse, beispielsweise Klettbänder, variabel verschlossen, wodurch der Kompressionsdruck der individuellen Anatomie des menschlichen Beines und den Bedürfnissen der individuell ausgeprägten Krankheitspartien anpassbar ist (DE 42 30 165 A1).

Die Wirkung all dieser therapeutischen Strümpfe basiert darauf, dass das verwendete Gewebe eine Kompressionskraft auf den Körper bzw. die Gliedmaßen ausübt. Die Ausübung von Kompressionskräften ist jedoch nicht optimal wirksam zur Verhinderung und/oder Verringerung der Ödembildung. Ein weiterer Nachteil dieser Orthesen besteht in der geringen Akzeptanz durch die Patienten. Die Ursachen liegen sowohl in der aufwändigen Prozedur des Anlegens als auch in der hohen körperlichen Belastung und dem unbequemen Tragekomfort. Da das verwendete Material eine bestimmte Eigenelastizität aufweisen muss, werden in der Regel elastische Fasern mit einem hohen Anteil an synthetischen Fasern verwendet, die bei Personen mit empfindlicher Haut zu Unannehmlichkeiten führen können und bei Personen mit einer totalen Hautunverträglichkeit bei synthetischen Geweben gar nicht angewendet werden können.

Zur Erleichterung des Anziehens einer schlauchförmigen Bandage, ohne dass bei nach dem Anlegen deren therapeutische Wirkung verlorengeht, ist es bekannt, die Bandage aus elastischen und wesentlich weniger elastischen Teilen herzustellen. In einer ersten Ausführung ist die im Wesentlichen aus einem weniger elastischen Teil bestehende Bandage mit einem Längsstreifen aus einem Material mit höherer Elastizität versehen, der mittels in Längsrichtung unelastischer und an dem unelastischen Teil befestigter Bänder so überbrückt wird, dass dessen Elastizität zumindest eingeschränkt werden kann. Das freie Ende der unelastischen Bänder ist durch eine auf der gegenüberliegenden Seite an dem unelastischen Teil befestigte Schlaufe gezogen und ist beispielsweise mittels eines Klettverschlusses mit sich selbst arretierbar.

Dadurch kann die wirksame Breite des elastischen Streifens verändert werden. Der elastische Längsstreifen kann in das Teil mit der geringeren Elastizität eingewebt oder eingewirkt sein.

In einer zweiten Ausführung sind jeweils paarweise ein Teil mit geringer Elastizität und ein Teil mit hoher Elastizität über die Länge der Bandage abwechselnd einander gegenüberliegend angeordnet. Jeweils die Teile mit der hohen Elastizität sind mit unelastischen Bändern überbrückt, die in gleicher Weise wie bei der o. g. ersten Variante an dem Teil mit der geringeren Elastizität befestigt sind (EP 0762 858 B1). Diese Bandage ist lediglich zur Stützung von Gelenken vorgesehen. Eine Anwendung zur Prophylaxe oder Behandlung von Störungen des Venen- und/oder Lymphsystems durch Entstauung ist nicht vorgesehen. Im Fall einer Anwendung dieser Bandage für eine derartige Behandlung würden die gleichen Nachteile wie bei den oben beschriebenen Bandagesystemen auftreten, da sie eine undifferenzierte Kompressionskraft auf den Körper oder die Gliedmaßen ausübt.

Bekannt sind ferner eine Vielzahl von Kompressionsbekleidungen, Funktionsstrümpfen, Kompressionsorthesen und Kompressionshilfen, die Zonen mit unterschiedlicher Dehnbarkeit und/oder Druckgradienten aufweisen. Diese Zonen unterschiedlicher Dehnbarkeit und/oder Druckgradienten erstrecken sich in Umfangsrichtung der Gliedmaßen und umschließen diese vollumfänglich (WO 2017213792 A1, AU 2013237693 B2, EP 1885310 B1, EP 2348902 B1, EP 0 927 014 B1). In Einzelfällen sind sie in Längsrichtung der Gliedmaßen geteilt ausgeführt und mit einstellbaren Verschlüssen versehen (AU 2013237693 B2). Die Wirkung dieser Orthesen besteht ebenfalls in der Ausübung von Kompressionskraft zur Entstauung der Ödeme bzw. zur Verhinderung der Ödembildung, die jedoch die gewünschte Entstauung in der Regel nicht, zumindest aber nicht vollständig erbringen, durch die hohe körperliche Belastung eine Einschränkung in der Beweglichkeit zu Folge haben und aufwändig im Anlegen und Abnehmen sind.

Der Erfindung liegt somit die Aufgabe zugrunde, ein Bandagesystem zu entwickeln, dessen Entstauungsfunktion eine hohe Wirksamkeit sowohl im Hinblick auf eine Verringerung vorhandener Ödeme als auch hinsichtlich der Prophylaxe, also der vorbeugenden Verhinderung von Störungen des Venen- und/oder Lymphsystems, besitzt.

Außerdem soll das Bandagesystem einen hohen Tragekomfort aufweisen und einfach handhabbar sein.

Die Aufgabe wird erfindungsgemäß durch die kennzeichnenden Merkmale des Anspruchs 1 gelöst.

### Die Erfindung und ihre Vorteile

Das erfindungsgemäße Bandagesystem ermöglicht eine hoch wirksame Entstauungsfunktion, ohne dass dazu das Gewebe des Bandagesystems eine Kompressionskraft auf den menschlichen Körper oder die Gliedmaßen aufbringen muss. Diese kompressionslose Entstauung wird dadurch erreicht, dass die Dehnungsrichtung der Gewebebereiche relativer Dehnbarkeit längs zur Arbeitsrichtung der Muskulatur des Körpers oder der Gliedmaße und somit quer zum Spaltlinienverlauf der Haut verläuft. Dadurch arbeitet das Bandagesystem richtungsorientiert.

Demgegenüber verlaufen die nicht dehnbaren Gewebebereiche in etwa in Richtung der Spaltlinien der Haut, d. h. quer zur Längsrichtung der Extremität bzw. Arbeitsrichtung der Muskulatur. Die Gewebebereiche mit und ohne Dehnbarkeit sind in Arbeitsrichtung der Muskulatur des Körpers oder der Gliedmaßen nacheinander abwechselnd angeordnet, so dass über die Länge des Bandagesystems eine bestimmte Anzahl von streifenförmigen Gewebebereichen abwechselnd mit und ohne Dehnbarkeit angeordnet sind. Die Breite der nicht dehnbaren Gewebebereiche wird individuell an den Patienten angepasst. Sie richtet sich nach den Schwellungen des Körperteils. Je größer die Schwellung ist, umso breiter wird der nicht dehnbare Gewebebereich ausgelegt.

Zur Anpassung des Bandagesystems an unterschiedliche Dicken von Körpern und/oder Gliedmaßen sowie an zurückgehende Ödeme ist das Bandagesystem in seinem Umfang einstellbar. Dies wird dadurch erreicht, dass die nicht dehnbaren, ringförmig verlaufenden Gewebebereiche des Bandagesystems nicht durchgehend geschlossen sind, den Körper oder die Extremität also nicht vollumfänglich umlaufen. Die Unterbrechung der nicht dehnbaren Gewebebereiche befindet sich bei Bandagesystemen für Extremitäten vorteilhafterweise an deren Innenseite und bei Bandagesystemen für den Rumpf an dessen Vorderseite. In diesen unterbrochenen Gewebebereichen ist zur Gewährleistung der festen Anlage des Bandagesystems am Körper bzw. Körperteil ein einstellbares und/oder nachstellbares Verschlusssystem, z. B. ein Klettverschluss vorgesehen. Dadurch kann das Bandagesystem bei sich zurückbildenden Ödemen nachgezogen und so an die veränderte Körperform angepasst werden. Das dehnbare Gewebe kann jedoch in diesem Bereich durchaus eine geschlossene Hülle bilden, so dass der einstellbare Bereich also mit dem gleichen, in Arbeitsrichtung der Muskulatur des Körpers oder der Gliedmaße dehnbaren Gewebe versehen sein kann. Die dehnbaren Gewebebereiche des Bandagesystems bilden in dem Fall also insgesamt eine geschlossene Umhüllung des Körpers oder der Extremität.

Wie oben bereits erwähnt, geht von dem Gewebe des erfindungsgemäßen Bandagesystems keine Kompressionskraft auf die Haut des Körpers oder der Gliedmaßen aus. Das Bandagesystem erfüllt dabei die Aufgabe der bekannten Kompressionsstrümpfe, nämlich das venöse System zu stützen, um ein Verbleiben von Gewebewasser zu minimieren, jedoch erfüllt es diese Aufgabe, ohne dabei das Arbeiten des oberflächigen lymphatischen Systems zu beinträchtigen. Das Gewebe des erfindungsgemäßen Bandagesystems ersetzt dadurch die biomechanischen Eigenschaften der Haut, deren Dehnung in den beiden senkrecht zueinander verlaufenden Richtungen ebenfalls unterschiedlich ist. In Richtung ihrer Spaltlinie, also quer zur Längsrichtung der Extremitäten und damit zur Arbeitsrichtung der Muskulatur, ist die Haut wesentlich weniger dehnbar und hat somit in dieser Richtung eine höhere Spannung als in Arbeitsrichtung der Muskulatur, in der wiederum die Spannung wegen der größeren Dehnfähigkeit deutlich geringer ist.

Diese medizinisch-anatomisch wirkenden Vorteile erbringen aber auch wesentliche Vorteile bezüglich des Tragekomforts des neuen Bandagesystems. Wie oben bereits erläutert, übt das Gewebe des Bandagesystems durch die kompressionslose Entstauung keine, zumindest aber keine spürbaren Kompressionskräfte auf den menschlichen Körper oder die Gliedmaßen aus. Durch die damit verbundene deutlich reduzierte körperliche Belastung und geringere Einschränkung der Beweglichkeit der vom Bandagesystem umhüllten Körperbereiche und Gliedmaßen erhöht sich der Tragekomfort des Bandagesystems erheblich. Da die Erzeugung der Dehnbarkeit nicht an bestimmte synthetische Materialien gebunden ist, ist bei Verwendung natürlicher Fasern für die Herstellung der Gewebe, wie Wolle, Leinen u. a., auch eine bessere Hautverträglichkeit gegeben. Zudem ist die Herstellung der Gewebe aus derartigen Materialien für das erfindungsgemäße Bandagesystem kostengünstiger, da die Fasern keine Eigenelastizität besitzen müssen, so dass an die für die Herstellung der Gewebe eingesetzten Maschinen keine besonderen Anforderungen gestellt werden müssen, wie das beispielsweise bei der Verarbeitung von elastischen synthetischen Fasern der Fall ist. Seine relative Dehnbarkeit, die allein der vollständigen Anlage des Bandagesystems am Körper und/oder den Gliedmaßen dient, erhält das Gewebe durch die Verarbeitung des selbst keine oder keine wesentliche Eigenelastizität aufweisenden Fadens durch den Web-, Wirk- oder Strickvorgang. Infolge all dieser positiven Eigenschaften des Gewebes kann auch davon ausgegangen werden, dass das erfindungsgemäße Bandagesystem von den Patienten besser angenommen werden wird als herkömmliche Bandagesysteme.

Bei einer vorteilhaften Ausgestaltung der Erfindung ist die Breite der nicht dehnbaren Gewebebereiche des Gewebes größer als die Breite der Gewebebereiche mit relativer Dehnbarkeit. Dadurch wird eine verbesserte äußere Stützung der umschlossenen Körperbereiche quasi als Ersatz der in diesen Körperbereichen zeitweise oder unwiederbringlich verloren gegangenen Stützkraft der Haut erreicht. Außerdem werden Einschnürungen an den von den nicht dehnbaren Gewebebereichen berührten Körperbereichen verhindert.

Bei einer zusätzlichen vorteilhaften Ausgestaltung der Erfindung gehen im Bereich von Gelenken die nicht dehnbaren Gewebebereiche strahlenförmig von der Gelenkachse und die Gewebebereiche mit relativer Dehnbarkeit von den um die Gelenkachse herum angeordneten Achspunkten aus. Die Gewebebereiche mit relativer Dehnbarkeit füllen die keilförmigen Zwischenräume zwischen den nicht dehnbaren Gewebebereichen aus. Dadurch wird im Bereich von Gelenken eine sichere Anlage des Bandagesystems an dem Gelenk erreicht und eine freie, bequeme Beweglichkeit der Gliedma-ßen ohne Verrutschen des Bandagesystems gewährleistet.

In einer ebenfalls vorteilhaften Ausgestaltung der Erfindung ist im Bereich außerhalb der Gelenke die Innenseite der nicht dehnbaren Gewebebereiche mit kleinen, zueinander beabstandeten Erhöhungen aus demselben Gewebe versehen. Dadurch wird dem Abfluss der Lymphe eine zusätzliche Unterstützung gegeben.

In einer diesbezüglich besonders vorteilhaften Ausgestaltung der Erfindung verlaufen diese Erhöhungen in Abflussrichtung der Territorialgebiete des Lymphsystems über die gesamte Breite der nicht dehnbaren Gewebebereiche. Dadurch wird dem Abfluss der Lymphe gleich die natürliche Richtung gegeben.

Die Gewebebereiche unterschiedlicher Dehnbarkeit können beispielsweise durch unterschiedliche Webtechniken oder die Verwendung unterschiedlich dehnbarer Materialien innerhalb eines Arbeitsganges erreicht werden. So ist es in einer vorteilhaften Ausgestaltung der Erfindung beispielsweise möglich, die nicht dehnbaren Gewebebereiche aus in das dehnbare Gewebe eingearbeiteten durch Muskelkraft nicht dehnbaren, jedoch biegsamen Materialien herzustellen. Andererseits ist es aber auch möglich, die nicht dehnbaren Gewebebereiche durch das Einarbeiten von durch Muskelkraft nicht dehnbarer Fäden über die vorgesehene Breite dieser Gewebebereiche in das dehnbare Gewebe zu erzeugen. Dies kann vorteilhafterweise während der Herstellung des dehnbaren Gewebes erfolgen. Bei diesem Herstellungsprozess entsteht somit das fertige Gewebe des Bandagesystems quasi in einem Arbeitsgang. Es ist aber auch möglich, in ein fertiges dehnbares Gewebe nachträglich durch Einarbeiten von durch Muskelkraft nicht dehnbaren Fäden die nicht dehnbaren Gewebebereiche in der erforderlichen Breite an den gewünschten Positionen zu erzeugen.

Weitere Vorteile und vorteilhafte Ausgestaltungen der Erfindung sind der nachfolgenden Beschreibung, den Ansprüchen und den Zeichnungen entnehmbar.

### Zeichnung

Ein bevorzugtes Ausführungsbeispiel des erfindungsgemäßen Bandagesystems ist in Form eines Entstauungsstrumpfes in den Zeichnungen dargestellt und wird im Folgenden näher erläutert. Es zeigen
- Fig. 1: eine prinzipmäßige Darstellung eines an ein Bein angelegten erfindungsgemäßes Entstauungsstrumpfes,
- Fig. 2: einen vergrößerten Ausschnitt aus dem Entstauungsstrumpf im Bereich seines oberen Endes,
- Fig. 3: einen vergrößerten Ausschnitt des Entstauungsstrumpfes im Bereich des Knies,
- Fig. 4: einen vergrößerten Ausschnitt des Entstauungsstrumpfes im Bereich oberhalb des Sprunggelenks des Beins,
- Fig. 5: einen vergrößerten Ausschnitt des Entstauungsstrumpfes im Bereich des Knies bei angewinkeltem Knie,
- Fig. 6: eine spezielle Ausführung der nicht dehnbaren Gewebebereiche des Entstauungsstrumpfes und
- Fig. 7: einen vergrößerten Ausschnitt des Entstauungsstrumpfes im Bereich seines Verschlusses.

### Beschreibung der Ausführungsbeispiele

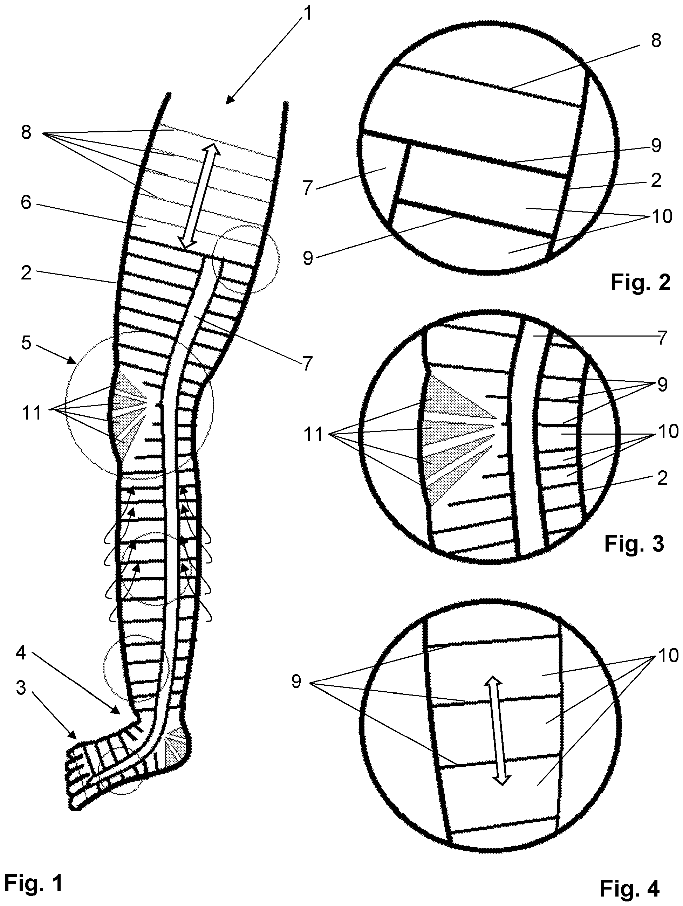

Fig. 1 zeigt einen an ein rechtes Bein 1 angelegten erfindungsgemäßen Entstauungsstrumpf 2, der von dem Zehengrundgelenk 3 des Fußes über dessen Sprunggelenk 4, das Kniegelenk 5 bis unterhalb des Endes des Oberschenkelstumpfes 6 reicht. Die Fig. 2 bis 4 zeigen vergrößerte Ausschnitte an verschiedenen Stellen des Entstauungsstrumpfes 2. An seiner Innenseite weist der Entstauungsstrumpf 2 eine vom Zehengrundgelenk 3 bis zu dem Oberschenkelstumpf 6 reichende verschließbare Naht 7 auf. Im Bereich des Oberschenkelstumpfes 6 sind Spaltlinien 8 der Haut angedeutet, die quer zur Längsrichtung bzw. Arbeitsrichtung der Muskulatur, also im Wesentlichen in Umfangsrichtung des Beines 1 verlaufen. Die Arbeitsrichtung der Muskulatur ist im Bereich des Oberschenkelstumpfes 6 durch einen Doppelpfeil angedeutet. Fig. 2 zeigt dazu eine vergrößerte Darstellung aus dem Bereich des Überganges vom Entstauungsstrumpf 2 zum Oberschenkelstumpf 6. Die von außerhalb des Entstauungsstrumpfes 2 schräg nach oben in Richtung des Oberschenkelstumpfes weisenden gekrümmten Pfeile deuten die Abflussrichtung der Lymphflüssigkeit in dem Unterschenkel des Beines 1 an.

Der Entstauungsstrumpf 2 besteht aus einem das Bein 1 umhüllenden Gewebe, das abwechselnd dehnbare Gewebebereiche 9 und nicht dehnbare Gewebebereiche 10 aufweist, wobei die weiß dargestellten, nicht dehnbaren Gewebebereiche 10 deutlich breiter sind als die als dicke Volllinien gezeichneten dehnbaren Gewebebereiche 9. Beide Gewebebereiche 9, 10 verlaufen in Richtung der Spaltlinien 8 der Haut. In dem in Fig. 4 dargestellten Detailausschnitt oberhalb des Sprunggelenkes 4 ist die Richtung der Dehnbarkeit der dehnbaren Gewebebereiche 9 durch einen Doppelpfeil angedeutet.

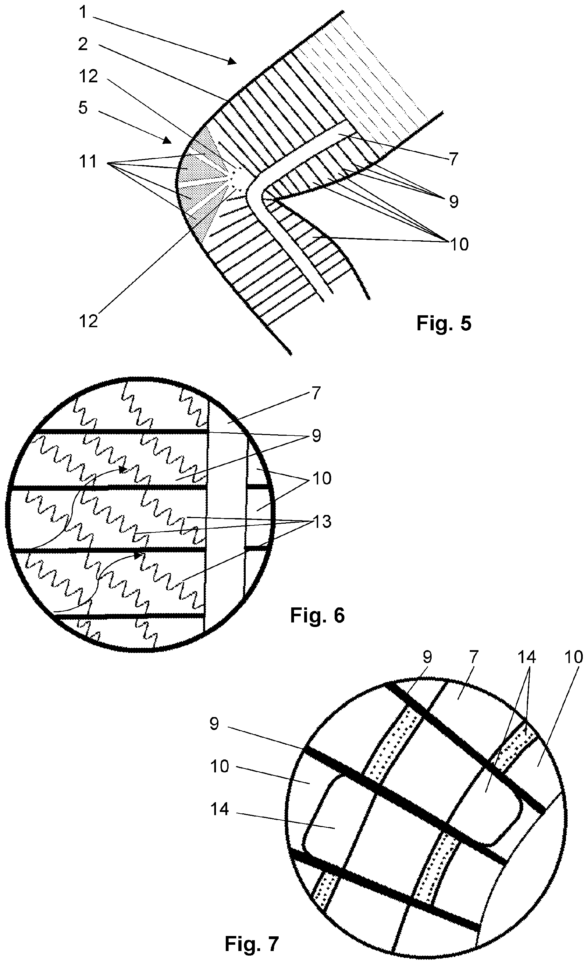

Zur Gewährleistung einer bequemen Beweglichkeit im Bereich der Gelenke, nämlich des Kniegelenks 5 und des Sprunggelenks 4, sowie einer sicheren Anlage des Bandagesystems im Bereich der Gelenke und um dadurch ein Verrutschen des Bandagesystems zu verhindern, sind zwischen den nicht dehnbaren Gewebebereiche 10 des Gewebes der Gelenke 4, 5 zusätzliche keilförmig ausgebildete dehnbare Gewebebereiche 11 vorgesehen. Diese weisen eine an die Funktion der Gelenke angepasste Ausrichtung derart auf, dass ihre zum Verschluss 7 hinweisenden Spitzen nicht von der zentralen Achse der Gelenke 4, 5, sondern von um diese herum verlaufenden Achspunkten 12 ausgehen, wie aus den Fig. 3 und 5 zu erkennen ist.

Fig. 6 zeigt einen vergrößerten Ausschnitt des erfindungsgemäßen Entstauungsstrumpfes 2 im Bereich der Wade des Beins mit einer seine therapeutische Wirkung verstärkenden Besonderheit an der Innenseite seiner nicht dehnbaren Gewebebereiche. Auf die Innenseite der nicht dehnbaren Gewebebereiche sind Erhöhungen 13 aus demselben Gewebe aufgebracht, die winklig zu den Gewebebereichen 9, 10 verlaufen und in den Territorialgebieten in Abflussrichtung der Lymphe wirksam werden. Auch in dieser Darstellung ist die Abflussrichtung der Lymphe durch die gekrümmten Pfeile angedeutet.

Anhand des in Fig. 7 gezeigten vergrößerten Ausschnitts des erfindungsgemäßen Entstauungsstrumpfes im Bereich des Zehengrundgelenkes 3 ist der Verschluss der Naht 7 beispielhaft mittels Klettverschlüssen 14 dargestellt. Es versteht sich von selbst, dass der Verschluss der Naht 7 über die gesamte Länge des Entstauungsstrumpfes 2 erfolgen muss.

Alle hier dargestellten Merkmale können sowohl einzeln als auch in beliebiger Kombination miteinander erfindungswesentlich sein.

### Bezugszahlenliste

- 1: Bein
- 2: Entstauungsstrumpf
- 3: Zehengrundgelenk
- 4: Sprunggelenk
- 5: Kniegelenk
- 6: Oberschenkelstumpf
- 7: Naht
- 8: Spaltlinie
- 9: Dehnbarer Gewebebereich
- 10: Nicht dehnbarer Gewebebereich
- 11: keilförmiger dehnbarer Gewebebereich
- 12: Achspunkt
- 13: Erhöhungen
- 14: Klettverschluss

## Patentansprüche

1. Bandagesystem mit Entstauungsfunktion zur Behandlung von Störungen des Lymph- und/oder Venensystems des menschlichen Körpers, bestehend aus einem Gewebe, das abwechselnd streifenförmige Bereiche mit relativer Dehnbarkeit und ohne Dehnbarkeit aufweist und den menschlichen Körper und/oder eine Gliedmaße zumindest teilweise umhüllt,
**dadurch gekennzeichnet,**
- **dass** die Dehnungsrichtung der Gewebebereiche relativer Dehnbarkeit (9) längs zur Arbeitsrichtung der Muskulatur des Körpers oder der Gliedmaße ausgerichtet ist,
- **dass** die Gewebebereiche ohne Dehnbarkeit (10) quer zur Arbeitsrichtung der Muskulatur verlaufen,
- **dass** die Gewebebereiche relativer Dehnbarkeit (9) und die Gewebebereiche ohne Dehnbarkeit (10) in Arbeitsrichtung der Muskulatur des Körpers oder der Gliedmaßen nacheinander abwechselnd angeordnet sind und
- **dass** das Bandagesystem in seinem Umfang einstellbar ist.

2. Bandagesystem nach Anspruch 1,
**dadurch gekennzeichnet,**
**dass** die Breite der nicht dehnbaren Gewebebereiche (10) größer ist als die Breite der Gewebebereiche (9) mit relativer Dehnbarkeit.

3. Bandagesystem nach Anspruch 1 oder 2,
**dadurch gekennzeichnet,**
**dass** im Bereich von Gelenken die nicht dehnbaren Gewebebereiche (10) strahlenförmig von der Gelenkachse und die Gewebebereiche mit relativer Dehnbarkeit von den um die Gelenkachse herum angeordneten Achspunkten (12) ausgehen und die keilförmigen Zwischenräume zwischen den nicht dehnbaren Gewebebereichen ausfüllen (10).

4. Bandagesystem nach Anspruch 1, 2 oder 3,
**dadurch gekennzeichnet,**
**dass** im Bereich außerhalb von Gelenken die Innenseite der nicht dehnbaren Gewebebereiche (10) mit zueinander beabstandeten Erhöhungen (13) aus demselben Gewebe versehen ist.

5. Bandagesystem nach Anspruch 4,
**dadurch gekennzeichnet,**
**dass** die Erhöhungen (13) in Abflussrichtung der Territorialgebiete des Lymphsystems über die gesamte Breite der nicht dehnbaren Gewebebereiche (10) verlaufen.

6. Bandagesystem nach Anspruch 5,
**dadurch gekennzeichnet,**
**dass** die nicht dehnbaren Gewebebereiche (10) an ihrem Umfang unterbrochen sind.

7. Bandagesystem nach Anspruch 6,
**dadurch gekennzeichnet,**
**dass** sich die Unterbrechung (7) der nicht dehnbaren Gewebebereiche (10) bei Bandagesystemen für Extremitäten an deren Innenseite und bei Bandagesystemen für den Rumpf an dessen Vorderseite befindet.

8. Bandagesystem nach Anspruch 5, 6 oder 7,
**dadurch gekennzeichnet,**
**dass** an der Unterbrechung (7) der nicht dehnbaren Gewebebereiche (10) ein einstellbares und/oder nachstellbares Verschlusssystem (14) angeordnet ist.

9. Bandagesystem nach einem der Ansprüche 1 bis 8,
**dadurch gekennzeichnet,**
**dass** die nicht dehnbaren Gewebebereiche (10) aus in dehnbares Gewebe eingearbeiteten durch Muskelkraft nicht dehnbaren, jedoch biegsamen Materialien bestehen.

10. Bandagesystem nach einem der Ansprüche 1 bis 8,
**dadurch gekennzeichnet,**
**dass** die nicht dehnbaren Gewebebereiche (10) aus in dehnbares Gewebe nebeneinander eingewebten, durch Muskelkraft nicht dehnbaren Fäden bestehen.

## Claims

1. A bandage system with a decongestive function for treating disorders of the lymphatic and/or venous system(s) of the human body, consisting of a textile fabric which has alternating relatively extensible strip-like regions and non-extensible strip-like regions and which at least partially surrounds the human body and/or a limb,
**characterised in that**
- the direction of extension of the relatively extensible textile fabric regions (9) are oriented along the working direction of the musculature of the body or the limb,
- the non-extensible textile-fabric regions (10) run transversely to the working direction of the musculature,
- the relatively extensible textile fabric regions (9) and the non-extensible textile fabric regions (10) are arranged alternately one after the other in the working direction of the musculature of the body or the limbs, and
- the circumference of the bandage system can be adjusted.

2. The bandage system as claimed in claim 1,
**characterised in that**
the width of the non-extensible textile fabric regions (10) is greater than the width of the relatively extensible textile fabric regions (9).

3. The bandage system as claimed in claim 1 or 2,
**characterised in that**
in the joint areas, the non-extensible textile fabric regions (10) are radially arranged, starting from the joint axis, and the relatively extensible textile fabric regions start from the axis points (12) which are arranged around said joint axis, filling up the wedge-like spaces between the non-extensible textile fabric regions (10).

4. The bandage system as claimed in claim 1, 2, or 3,
**characterised in that**
in areas other than joint areas, the inner surface of the non-extensible textile fabric regions (10) is provided with raised portions (13) spaced apart from each other and made of the same textile fabric.

5. The bandage system as claimed in claim 4,
**characterised in that**
the raised portions (13) in the drainage direction of the territorial regions of the lymphatic system run across the entire width of the non-extensible textile fabric regions (10).

6. The bandage system as claimed in claim 5,
**characterised in that**
the non-extensible textile fabric regions (10) are interrupted at their circumference.

7. The bandage system as claimed in claim 6,
**characterised in that**
for bandage systems to be used on the extremities, the interruptions (7) of the non-extensible textile fabric regions (10) are located at the inner side of said extremities, and for bandage systems to be used on the trunk, they are located at the front side of said trunk.

8. The bandage system as claimed in claim 5, 6, or 7,
**characterised in that**
an adjustable and/or re-adjustable closure system (14) is arranged at the interruption (7) of the non-extensible textile fabric region (10).

9. The bandage system as claimed in any one of claims 1 to 8,
**characterised in that**
the non-extensible textile fabric regions (10) consist of materials which are incorporated into extensible textile fabric and which are not extensible by muscular force but which are bendable.

10. The bandage system as claimed in any one of claims 1 to 8,
**characterised in that**
the non-extensible textile fabric regions (10) consist of threads which are woven into extensible textile fabric one next to the other and which are not extensible by muscular force.

## Revendications

1. Système de bandage avec fonction de décongestion destiné à traiter des troubles du système lymphatique et/ou veineux du corps humain, constitué d'un tissu qui présente en alternance des zones en forme de bande à extensibilité relative et sans extensibilité, et qui recouvre au moins partiellement le corps humain et/ou un membre,
**caractérisé en ce que**
- le sens d'extension des zones de tissu à extensibilité relative (9) est orienté de manière longitudinale au sens de travail de la musculature du corps ou du membre,
- les zones de tissu sans extensibilité (10) s'étendent de manière transversale au sens de travail de la musculature,
- les zones de tissu à extensibilité relative (9) et les zones de tissu sans extensibilité (10) sont disposées alternativement à la suite les unes des autres dans le sens de travail de la musculature du corps ou des membres et
- la circonférence dudit système de bandage peut être ajustée.

2. Système de bandage selon la revendication 1,
**caractérisé en ce que**
la largeur des zones de tissu non extensibles (10) est supérieure à la largeur des zones de tissu à extensibilité relative (9).

3. Système de bandage selon la revendication 1 ou 2,
**caractérisé en ce que**
dans la zone des articulations, les zones de tissu non extensibles (10) rayonnent à partir de l'axe d'articulation et les zones de tissu à extensibilité relative rayonnent à partir des points d'axe (12) disposés autour dudit axe d'articulation et remplissent les espaces en forme de coin formés entre les zones de tissu non extensibles (10).

4. Système de bandage selon la revendication 1, 2 ou 3,
**caractérisé en ce que**
dans la zone située à l'extérieur des articulations, la face intérieure des zones de tissu non extensibles (10) est pourvue de saillies (13) séparées les unes des autres qui sont réalisées dans le même tissu.

5. Système de bandage selon la revendication 4,
**caractérisé en ce que**
les saillies (13) s'étendent dans le sens d'écoulement des régions territoriales du système lymphatique, sur toute la largeur des zones de tissu non extensibles (10).

6. Système de bandage selon la revendication 5,
**caractérisé en ce que**
les zones de tissu non extensibles (10) sont interrompues au niveau de leur circonférence.

7. Système de bandage selon la revendication 6,
**caractérisé en ce que**
l'interruption (7) des zones de tissu non extensibles (10) se situe, dans le cas de systèmes de bandage pour extrémités, sur la face intérieure des extrémités et, dans le cas de systèmes de bandage pour le torse, sur la face avant du torse.

8. Système de bandage selon la revendication 5, 6 ou 7,
**caractérisé en ce que**
un système de fermeture (14) ajustable et/ou réajustable est disposé sur l'interruption (7) des zones de tissu non extensibles (10).

9. Système de bandage selon l'une quelconque des revendications 1 à 8,
**caractérisé en ce que**
les zones de tissu non extensibles (10) sont constituées de matériaux non extensibles sous l'action de la force musculaire, mais cependant souples, qui sont intégrés dans du tissu extensible.

10. Système de bandage selon l'une quelconque des revendications 1 à 8,
**caractérisé en ce que**
les zones de tissu non extensibles (10) sont constituées de fils non extensibles sous l'action de la force musculaire qui sont intégrés par tissage côte à côte dans du tissu extensible.
